# EUROPEAN PATENT APPLICATION

(11) **EP 4 438 092 A1**
(43) Date of publication of application: **02.10.2024**
(21) Application number: 24163172.0
(22) Date of filing: 13.03.2024
(51) Int. Cl.: A61M 16/01, A61M 16/10, A61M 16/08

(54) **SYSTEMS AND METHODS FOR ANESTHESIA MACHINES WITH VENTILATOR DRIVE GAS INSPIRATORY FLOW**

(30) Priority: 30.03.2023 US 202318193441
(71) Applicant: GE Precision Healthcare LLC, Waukesha, WI 53188 (US)
(72) Inventor: BAUM, Bradley, Madison, 53718 (US)
(74) Representative: Kilburn & Strode LLP

(57) **Abstract**

A method (800) for an anesthesia system (600) includes flowing ventilator drive gas through a bellowless plenum (502) separating a ventilator drive gas circuit (352) from an inspiratory gas circuit (500); and providing the ventilator drive gas flowing through the bellowless plenum (502) to an inspiratory port (518) of the inspiratory gas circuit (500).

## Description

### TECHNICAL FIELD

Embodiments of the subject matter disclosed herein relate to anesthesia machines, and more particularly, to anesthesia machines including electronic ventilators and bellowless breathing systems.

### BACKGROUND

Anesthesia machines often include a plurality of gas passages and other components adapted to flow gases to a subject from a gas mixer. The gas mixer may form a combination of gases such as oxygen, nitrogen, and/or air and may flow the gases individually or in combination to an inspiratory port for inhalation by a patient. Exhalation gases resulting from the patient may be returned to the anesthesia machine. Some anesthesia machines may include a bellows configured to drive the gases from the gas mixer and inspiratory circuit to the inspiratory port for inhalation by the patient, where compression of the bellows is provided by drive gases flowing to exterior surfaces of the bellows via a ventilator. Other anesthesia machines may include a bellowless inspiratory circuit, where the drive gases of the ventilator force the gases from the gas mixer and inspiratory circuit to flow to the inspiratory port but are maintained separate from gases of the gas mixer via a bellowless plenum. In addition to the previous descriptions, other anesthesia machines may offer auxiliary gas outputs which utilize the existing gas mixer. However, gas mixers of anesthesia machines may have a narrow range of available flow rates which may not be sufficient for some clinician applications such as intubation using a high flow nasal cannula. The current solution to this problem is to add separate hardware, either internal or external, to the anesthesia machine to achieve the flow rates needed for some clinical applications such as intubation using a high flow nasal cannula.

### SUMMARY

The current disclosure at least partially addresses one or more of the above identified issues by a method for an anesthesia system, comprising: flowing ventilator drive gas through a bellowless plenum separating a ventilator drive gas circuit from an inspiratory gas circuit; and providing the ventilator drive gas flowing through the bellowless plenum to either an inspiratory port of the inspiratory gas circuit or an auxiliary port.

In various embodiments, the ventilator drive gas may be one of a plurality of ventilator drive gases selectable via a drive gas selector. The selected ventilator drive gas may be mixed with gas from a gas mixer of the anesthesia machine to form an inspiratory mixture with a user-selected ratio of ventilator drive gas to mixer gas from the gas mixer. An advantage of providing ventilator drive gas to the inspiratory port or auxiliary port via the bellowless plenum over other approaches is that a high gas flow may be provided to the patient via a nasal cannula or other device, e.g., for patient intubation, without adding additional ventilators and/or additional gas mixers to the anesthesia machine. As a result, a cost and/or maintenance complexity of the anesthesia machine may be reduced, and increased functionality of the anesthesia machine may be provided via the high flow gas output for clinician operations such as patient intubation.

The above advantages and other advantages, and features of the present description will be readily apparent from the following Detailed Description when taken alone or in connection with the accompanying drawings. It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

Various aspects of this disclosure may be better understood upon reading the following detailed description and upon reference to the drawings in which:
FIG. 1 shows a perspective view of an example anesthesia machine according to an embodiment;
FIG. 2 shows a schematic diagram of an anesthesia machine according to an embodiment;
FIG. 3 shows a schematic diagram of a ventilator assembly of an anesthesia machine according to an embodiment;
FIG. 4 shows a schematic diagram of a gas mixer of an anesthesia machine according to an embodiment;
FIG. 5 shows a schematic diagram of an inspiratory circuit of an anesthesia machine including a bellowless plenum according to an embodiment;
FIG. 6 shows a schematic diagram of an anesthesia machine including the ventilator assembly of FIG. 3, the gas mixer of FIG. 4, the inspiratory circuit of FIG. 5 according to an embodiment, and a first ventilator drive gas flow path according to an embodiment;
FIG. 7 shows a schematic diagram of an anesthesia machine including the ventilator assembly of FIG. 3, the gas mixer of FIG. 4, the inspiratory circuit of FIG. 5 according to an embodiment, and a second ventilator drive gas flow path according to an embodiment;
FIG. 8 shows a flowchart illustrating a method for an anesthesia machine configured to flow gas from a ventilator drive gas source to an inspiratory port, according to an embodiment;
FIG. 9 shows a flowchart illustrating a method for selecting high gas flow mode parameters for an anesthesia machine configured to flow gas from a ventilator drive gas source to an inspiratory port; and
FIG. 10 shows a flowchart illustrating a method for providing selectable high gas flow mode parameters for an anesthesia machine configured to flow gas from a ventilator drive gas source to an inspiratory port.

The drawings illustrate specific aspects of the described systems and methods for anesthesia machines configured to flow gas from a ventilator drive gas source to an inspiratory port. Together with the following description, the drawings demonstrate and explain the structures, methods, and principles described herein. In the drawings, the size of components may be exaggerated or otherwise modified for clarity. Well-known structures, materials, or operations are not shown or described in detail to avoid obscuring aspects of the described components, systems, and methods.

### DETAILED DESCRIPTION

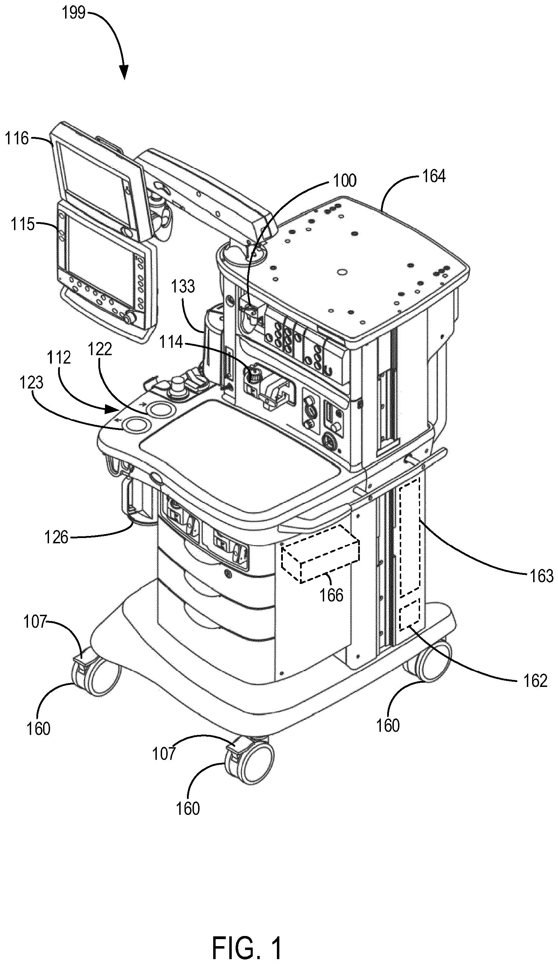

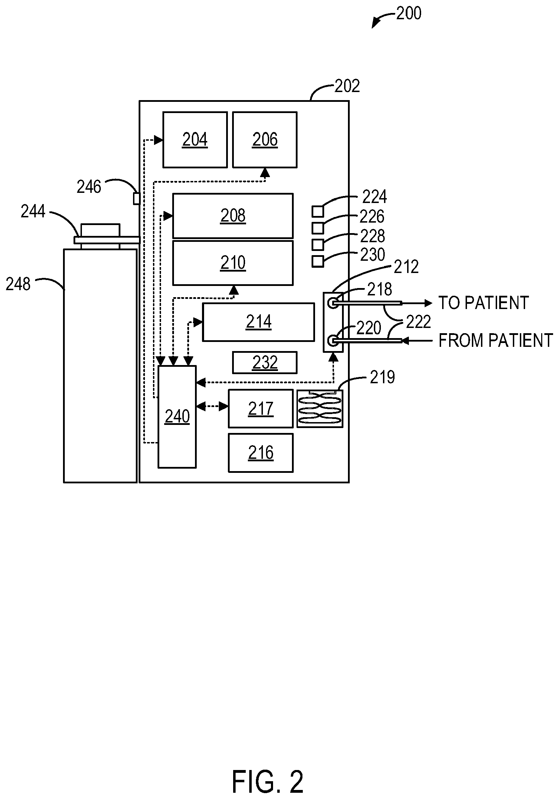

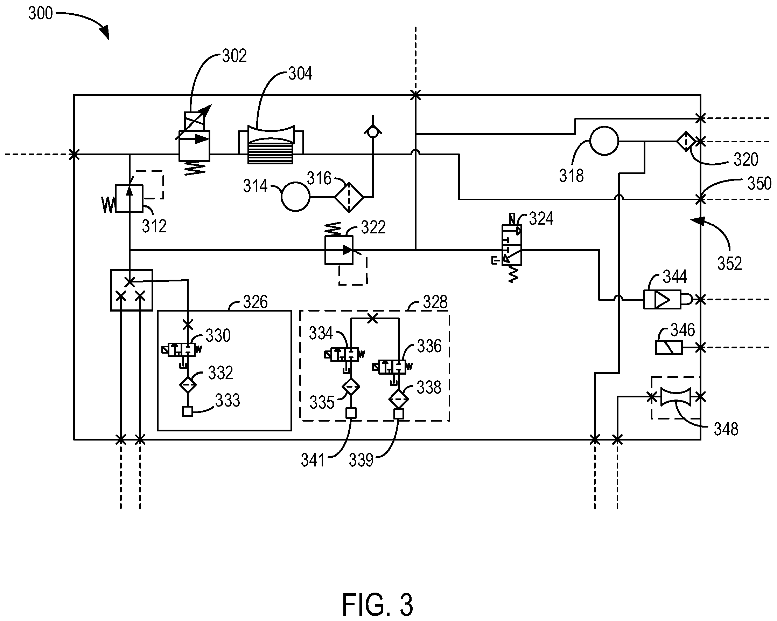

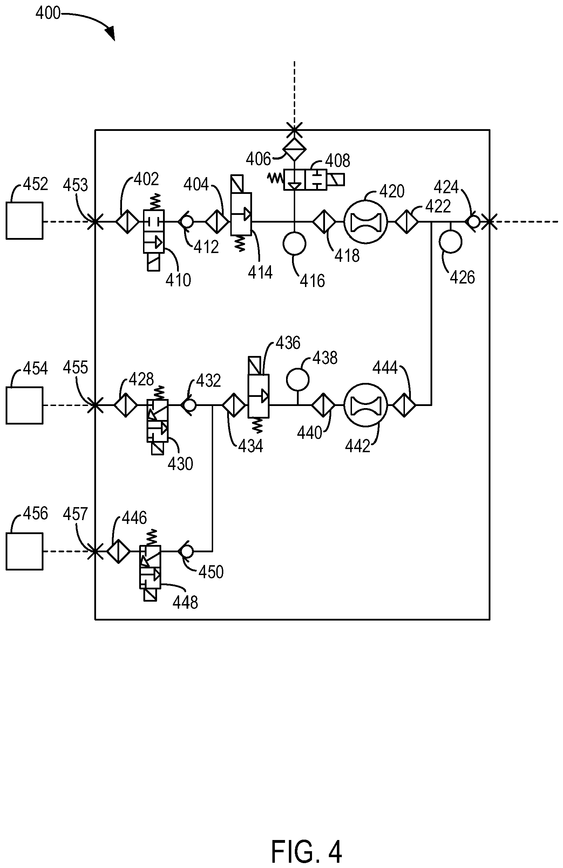

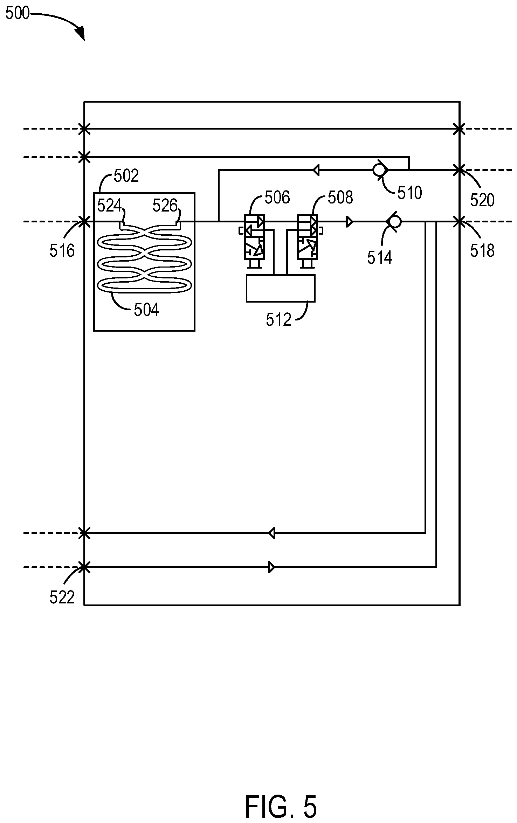

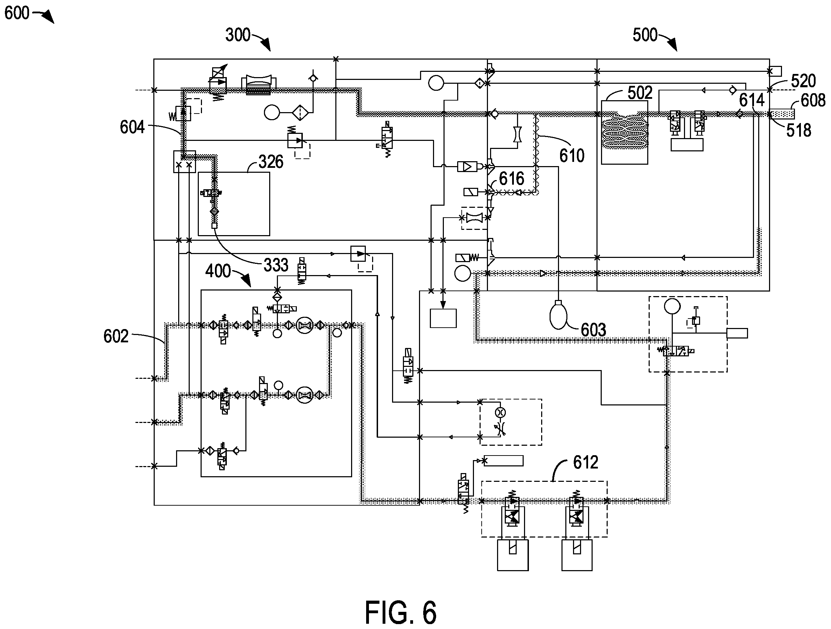

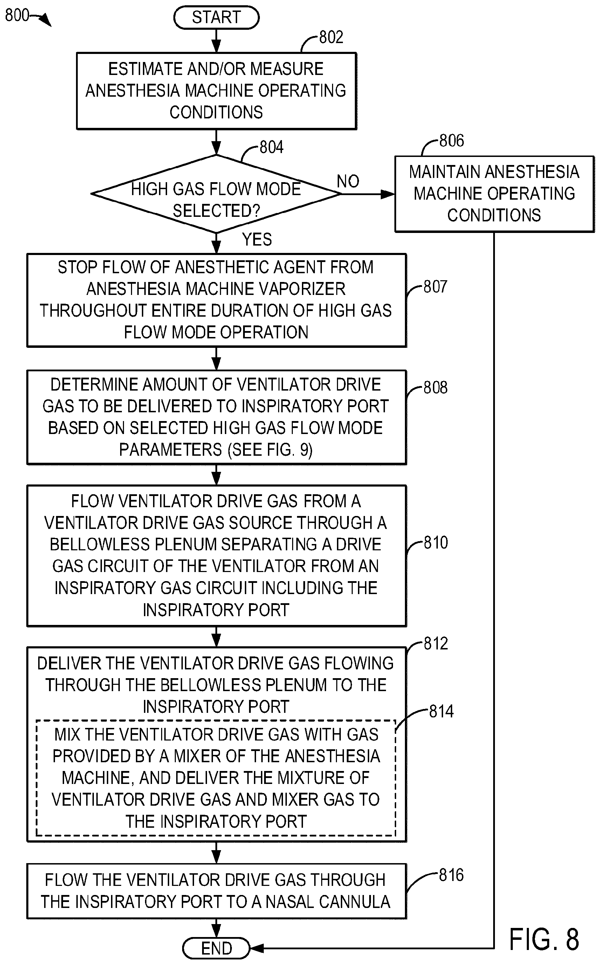

Systems and methods are disclosed for anesthesia machines including electronic ventilators and bellowless inspiratory circuits. An anesthesia machine, such as the anesthesia machine shown by FIG. 1, may be configured with one or more ventilator drive gas sources, such as the ventilator drive gas source shown by FIG. 2. The ventilator drive gas source may flow ventilator drive gas through the ventilator assembly, such as the ventilator assembly shown by FIG. 3, toward a bellowless plenum included within an inspiratory circuit of the anesthesia machine, such as the inspiratory circuit shown by FIG. 5. According to one or more embodiments, the ventilator drive gas flows through the bellowless plenum, as shown by FIGS. 6-7, and is provided to an inspiratory port of the inspiratory circuit for inhalation by a patient. In some embodiments, the ventilator drive gas provided to the inspiratory port may flow through a nasal cannula for inhalation by the patient. The ventilator drive gas may be combined with mixer gas from a gas mixer of the anesthesia machine, such as the gas mixer shown by FIG. 4, to form a mixture of ventilator drive gas and mixer gas in some embodiments, and the mixture may be provided to the inspiratory port for inhalation by the patient. As described herein and as shown by the methods of FIGS. 8-10, parameters of the ventilator drive gas (or mixture of ventilator drive gas and mixer gas) provided to the inspiratory port may be based on a configuration of the anesthesia machine and may be selected by a user of the anesthesia machine, such as a clinician. As a result, the anesthesia machine may provide a high gas flow for inhalation by the patient during various clinical operations such as patient intubation without configuring the anesthesia machine with additional ventilators and/or additional gas mixers, and a cost and/or maintenance complexity of the anesthesia machine may be reduced.

Referring to FIG. 1, an anesthesia machine 199 is shown from a side perspective view. The anesthesia machines described herein, such as anesthesia machine 199, may be referred to as anesthesia systems. Anesthesia machine 199 includes a frame 164 supported by casters 160, where the movement of the casters may be controlled (e.g., stopped) by one or more locks 107. In some examples, the frame 164 may be formed of a plastic material (e.g., polypropylene). In other examples, the frame 164 may be formed from a different type of material (e.g., metal, such as steel).

Anesthesia machine 199 also includes respiratory gas module 100, ventilator 112 (explained in more detail below), vaporizer 114 (explained in more detail below), anesthesia display device 115, and patient monitoring display device 116.

Vaporizer 114 may be in the form of a removable cassette such that a supply of anesthetic agent can be removed from the cassette. Different types of anesthetic agents can be supplied to the anesthesia machine by simply removing the cassette and replacing it with a different cassette for a different anesthetic agent. The vaporizer 114 may include a housing having a drug reservoir that contains a supply of anesthetic agent to be delivered to a patient. The drug reservoir may have a discharge opening formed in a back wall of the housing that is configured to receive a discharge tube (not shown), which may be part of the anesthesia machine, forming a gas-tight seal for delivery of anesthetic vapor to a patient.

Vaporizer 114 may include at least one absorbent wick within the reservoir. A passageway allows fresh gas from a gas source to pass to the absorbent wick(s). Vaporized liquid from the absorbent wick(s) and accompanying fresh gas may flow to the back of the drug reservoir and out the discharge opening in the housing. A lower portion of the drug reservoir may contain the liquid anesthetic agent and an upper portion of the reservoir may contain the vaporized anesthetic agent and breathing gases. During operation, a combination of temperature and pressure affect the liquid anesthetic agent and cause it to vaporize into the breathing gases. The gases carrying the vaporized agent are then discharged through the discharge opening.

A rear of the anesthesia machine 199 may include one or more pipeline connections to facilitate coupling of the anesthesia machine to pipeline gas sources. The pipeline gas sources may be ventilator drive gas sources, in some embodiments. The rear of the anesthesia machine may include a cylinder yoke, via which one or more gas-holding cylinders may be coupled to the anesthesia machine. In some embodiments, the gas-holding cylinders may be ventilator drive gas sources. Thus, through the pipeline connection and/or cylinder connections, gas (e.g., ventilator drive gas) may be provided to the anesthesia machine, where the gas may include but is not limited to fresh gas, oxygen, and nitrous oxide.

In some embodiments, one or more of the pipeline connections and/or gas cylinders may be mixer gas sources configured to provide gas to a gas mixer 163 of the anesthesia machine (shown schematically in FIG. 1). The gas that enters the anesthesia machine may mix with the vaporized anesthetic agent at the vaporizer and may be supplied to a patient via the gas mixer. In some embodiments, the rear of the anesthesia machine may also include a serial port, a collection bottle connection, cylinder wrench storage, anesthesia gas scavenging system, main power inlet, system circuit breaker, equipotential stud, outlet circuit breaker, and isolated electrical outlet.

The ventilator 112 may include an expiratory check valve 122, inspiratory check valve 123, absorber canister 126, and bellowless plenum 133. When a patient breathing circuit (which may be referred to herein as an inspiratory circuit) is coupled to the ventilator, the breathing gases (e.g., fresh gas, oxygen, and/or nitrous oxide mixed with vaporized anesthetic agent) exit the machine from an inspiratory port (which may be positioned at the same location as the inspiratory check valve 123) and travel to a patient. The inspiratory port may be referred to herein as a patient inspiratory port. During standard ventilation, expiratory gases from the patient may re-enter the anesthesia machine via an expiratory port (which may be positioned at the same location as the expiratory check valve 122), where the carbon dioxide may be removed from the expiratory gases via the absorber canister 126. However, during some clinician applications such as intubation using a high flow nasal cannula, an exhalation valve of the anesthesia machine may be maintained in a closed position and the patient may exhale the inhaled gases to atmosphere.

During operation of the vaporizer 114, an operator (e.g., an anesthesiologist, clinician, etc.) may adjust an amount of vaporized anesthetic agent that is supplied to a patient by adjusting a flow rate of gases from the gas source(s) of the gas mixer (e.g., one or more of the gas pipelines) to the vaporizer. The flow rate of the gases from the gas mixer to the vaporizer may be adjusted by the operator via adjustment of one or more flow adjustment devices. For example, the flow adjustment devices may include analog and/or digital adjustment dials and/or other user input devices configured to actuate one or more flow control valves of the anesthesia machine 199. In one example, a first flow control valve may be positioned between the gas source(s) and the gas mixer and may be actuatable via the flow adjustment devices to a fully opened position, a fully closed position, and a plurality of positions between the fully opened position and the fully closed position. Gases flowing from the gas mixer may mix and/or converge with vaporized anesthetic agent within the vaporizer and may flow to the inspiratory port for inhalation by the patient.

The anesthesia machine may additionally include one or more bypass valves configured to bypass gases from the gas source(s) around the vaporizer 114. The bypass valves may enable a first portion of gases flowing from the gas source to flow directly from the gas source to the inspiratory port, and a second portion of gases flowing from the gas source may flow through the vaporizer 114 to mix with the vaporized anesthetic agents prior to flowing to the inspiratory port. By adjusting a ratio of an amount of gases flowing to the port via the bypass valves relative to an amount of gases flowing to the port via the vaporizer 114, the operator may control a concentration of vaporized anesthetic agent in gases at the inspiratory port.

Further, the adjustments described above may be facilitated at least in part based on output from the respiratory gas module 100. Respiratory gas module 100 may be configured to measure various parameters of the gases exiting the vaporizer and/or being provided to the patient. For example, respiratory gas module 100 may measure the concentrations of carbon dioxide, nitrous oxide, and anesthesia provided to the patient. Further, respiratory gas module 100 may measure respiration rate, minimum alveolar concentration, patient oxygen, and/or other parameters. The output from the respiratory gas module 100 may be displayed via a graphical user interface displayed on a display device (e.g., device 115 and/or 116) and/or used by the controller to provide closed-loop feedback control of the amount of anesthesia provided to the patient.

Ventilator 112 may optionally be coupled to a breathing circuit (not shown) including a plurality of tubes (e.g., gas passages). The breathing circuit may be coupled between an airway of a patient (e.g., via a breathing mask positioned to enclose the mouth and/or nose of the patient) and the inspiratory port. Gases (e.g., oxygen, or a mixture of oxygen and vaporized anesthetic agents from vaporizer 114) may flow from the port, through the breathing circuit, and into the airway of the patient, where the gases are absorbed by the lungs of the patient. By adjusting the concentration of vaporized anesthetic agent in the gases as described above, the operator may adjust an amount by which the patient is anesthetized.

The ventilator 112 may be an electronic ventilator controlled via an electronic controller 162, and operation of the ventilator may be adjusted according to user-selected parameters. For example, during conditions in which a user (e.g., a clinician) selects a high flow mode of the anesthesia machine, the controller 162 may control the ventilator 112 to flow ventilator drive gas from one or more ventilator drive gas sources through the bellowless plenum to the inspiratory port. However, during conditions in which the high flow mode is not selected, the controller 162 may controller the ventilator 112 to provide ventilator drive gases to the bellowless plenum to drive mixer gases flowing from the gas mixer to the inspiratory port and without flowing the ventilator drive gases to the inspiratory port. The flow of ventilator drive gases within the bellowless plenum may be a laminar flow, and the flow of mixer gases within the bellowless plenum may be a laminar flow, with the laminar flow of the ventilator drive gases being separate from the laminar flow of the mixer gases.

During conditions in which the breathing circuit is coupled to the airway, anesthetic agent and/or fresh gases may flow into the airway (e.g., be inhaled by the patient) via the inspiratory check valve 123. For example, inspiratory check valve 123 may open automatically (e.g., without input or adjustment by the operator) in response to inhalation of the patient, and may close automatically in response to exhalation of the patient. Similarly, the expiratory check valve 122 may open automatically in response to exhalation of the patient, and may close automatically in response to inhalation of the patient.

In some examples, the operator may alternately and/or additionally control one or more operating parameters of the anesthesia machine 199 via electronic controller 162 of the anesthesia machine 199 (shown schematically by FIG. 1). Controller 162 includes a processor operatively connected to a memory. The memory may be a non-transitory computer-readable medium and may be configured to store computer executable code (e.g., instructions) to be processed by the processor in order to execute one or more routines as described herein. The memory may also be configured to store data received by the processor. Controller 162 may be communicatively (e.g., wired or wirelessly) coupled to one or more external or remote computing devices, such as a hospital computing system, and may be configured to send and receive various information, such as electronic medical record information, procedure information, and so forth. In some embodiments, controller 162 may control operation of the flush module 166 in order to perform one or more cleaning cycles, disinfecting cycles, flush cycles, etc. of a gas path of the anesthesia machine 199.

The controller receives signals from the various sensors of the anesthesia machine 199 and employs the various actuators of the anesthesia machine 199 to adjust operation of the anesthesia machine 199 based on the received signals and instructions stored on the memory of the controller. For example, the flow of gases to the inspiratory port may be controlled via an input device (e.g., keyboard, touchscreen, etc.) coupled to the electronic controller of the anesthesia machine 199. As one example, a user may input a selection via the input device (which may be referred to herein as a user interface device) in order to adjust the anesthesia machine from operating in the high flow mode in which ventilator drive gas is provided through the bellowless plenum to the inspiratory port, to operating in a lower flow mode in which ventilator drive gas does not flow to the inspiratory port and gas for inhalation by the patient is provided by the gas mixer. The controller may be electrically coupled to display device 115 and/or 116 in order to display operating parameters of the anesthesia machine 199. The controller may receive signals (e.g., electrical signals) via the input device and may adjust operating parameters of the anesthesia machine 199 in response (e.g., responsive) to the received signals. For example, the operator may input a desired flow rate of gases (e.g., oxygen) flowing from the gas mixer to the patient and/or vaporizer 114.

A corresponding valve position of one or more valves of the anesthesia machine (e.g., a position of one or more bypass valves, as described above) may be empirically determined and stored in a predetermined lookup table or function on the controller. For example, the controller may receive the desired flow rate of gases via the input device and may determine an amount of opening of the one or more bypass valves corresponding to the desired flow rate based on the lookup table, with an input being the desired flow rate and an output being the valve position. The controller may transmit an electrical signal to an actuator of the valve in order to adjust the valve position. In some examples, the controller may compare the desired flow rate of gases to a measured flow rate of gases as measured by a flow rate sensor (e.g., an inspiratory flow sensor).

Controller 162 is shown in FIG. 1 for illustrative purposes, and it is to be understood that controller 162 may be located internally of anesthesia machine 199 and thus may not be visible externally on anesthesia machine. Controller 162 may include multiple devices/modules that may be distributed across anesthesia machine 199.

In the embodiment described above, the gas mixer of the anesthesia machine may be the only gas mixer of the anesthesia machine, and the ventilator of the anesthesia machine may be the only ventilator of the anesthesia machine. For example, according to the embodiments described herein (e.g. the embodiment of the anesthesia machine shown by FIG. 1), the high gas flow mode of the anesthesia machine may be selected to provide ventilator drive gas to the inspiratory port via the ventilator and the bellowless plenum without additional ventilators.

Referring to FIG. 2, another example anesthesia machine 200 is shown. The anesthesia machine 200 is shown schematically by FIG. 2. Anesthesia machine 200 includes a frame (or housing) 202. In some embodiments, frame 202 may be supported by casters, where the movement of the casters may be controlled (e.g., stopped) by one or more locks. In some examples, the frame 202 may be formed of a plastic material (e.g., polypropylene). In other examples, the frame 202 may be formed of a different type of material (e.g., metal, such as steel).

Anesthesia machine 200 also includes an anesthesia display device 204, a patient monitoring display device 206, a respiratory gas module 208, one or more patient monitoring modules, such as a patient monitoring module 210, a ventilator 212, an anesthetic vaporizer 214, and an anesthetic agent storage bay 216. Anesthesia machine 200 may further include a main power indicator 224, a system activation switch 226 (which, in one example, permits gas flow when activated), an oxygen flush button 228, and an oxygen control 230. Anesthetic vaporizer 214 may vaporize the anesthetic agent and combine the vaporized anesthetic agent with one or more medical grade gases (e.g., oxygen, air, nitrous oxide, or combinations thereof), which may then be delivered to a patient.

Anesthesia machine 200 may additionally include an integrated suction, an auxiliary oxygen flow control, and various other components for providing and/or controlling a flow of the one or more medical grade gases to the patient. In the embodiment shown, anesthesia machine 200 includes one or more pipeline connections 246 to facilitate coupling of the anesthesia machine to pipeline gas sources. Additionally, anesthesia machine 200 includes a cylinder yoke 244, via which one or more gas-holding cylinders 248 may be coupled to the anesthesia machine. Ventilator drive gas sources of the anesthesia machine may include one or more of the pipeline gas sources and/or gas-holding cylinders. Further, mixer gas sources of the anesthesia machine may include one or more of the pipeline gas sources and/or gas-holding cylinders, which may be maintained separately from the ventilator drive gas sources. Thus, through the pipeline connection and/or cylinder connections, gas may be provided to the anesthesia machine, where the gas may include (but is not limited to) medical air, oxygen, nitrogen, and nitrous oxide. The gas that enters the anesthesia machine may mix with the vaporized anesthetic agent at the anesthetic vaporizer 214, as described above, before being supplied to a patient via an inspiratory port 218. In particular, gases provided from the mixer gas sources to a gas mixer 217 may flow from the mixer to the anesthetic vaporizer 214, where the gases may mix with vaporized anesthetic agent and may flow to the inspiratory port 218 for inhalation by the patient. The mixer gases flowing from the gas mixer 217 may be driven toward the inspiratory port 218 via the ventilator 212, where the ventilator 212 may provide ventilator drive gases to bellowless plenum 219 separating the ventilator 212 from the gas mixer 217. The flow of ventilator drive gases to the bellowless plenum may be controlled by the controller 240 in order to drive the mixer gases toward the inspiratory port (e.g., via a pressure of the ventilator drive gases). The anesthesia machine may also include a serial port, a collection bottle connection, and a cylinder wrench storage area. Further, in some embodiments, the anesthesia machine may include an anesthesia gas scavenging system 232.

The ventilator 212 may include an expiratory check valve at an expiratory port 220, an expiratory flow sensor at the expiratory port 220, an inspiratory check valve at inspiratory port 218, an inspiratory flow sensor at the inspiratory port 218, an absorber canister, a manual bag port, a ventilator release, an adjustable pressure-limiting valve, a bag/vent switch, and a bellowless plenum. When a patient breathing circuit is coupled to the ventilator 212, breathing gases (e.g., air, oxygen, and/or nitrous oxide mixed with vaporized anesthetic agent) exit the anesthesia machine from the inspiratory port 218 and travel to the patient. Expiratory gases from the patient may re-enter the anesthesia machine in some conditions (e.g., low flow conditions) via the expiratory port 220, where carbon dioxide may be removed from the expiratory gases via the absorber canister.

During operation of the anesthetic vaporizer 214, an operator (e.g., an anesthesiologist) may adjust an amount of vaporized anesthetic agent that is supplied to the patient by adjusting a flow rate of gases from the gas source(s) (e.g., the pipeline gas supply) to the vaporizer. The flow rate of the gases from the gas source to the vaporizer may be adjusted by the operator via adjustment of one or more flow adjustment devices. For example, the flow adjustment devices may include analog and/or digital adjustment dials and/or other user input devices configured to actuate one or more flow control valves of anesthesia machine 200. In some embodiments, a first flow control valve may be positioned between the gas source(s) and the anesthetic vaporizer 214 and may be actuatable via the flow adjustment devices to a fully opened position, a fully closed position, and a plurality of positions between the fully opened position and the fully closed position.

Anesthesia machine 200 may additionally include one or more valves configured to bypass gases from the gas source(s) around the anesthetic vaporizer 214. The valves may enable a first portion of gases to flow directly from the gas source to the inspiratory port 218 and a second portion of gases to flow from the gas source through the anesthetic vaporizer 214 to mix with the vaporized anesthetic agents prior to flowing to the inspiratory port 218. By adjusting a ratio of the first portion of gases relative to the second portion of gases, the operator may control a concentration of vaporized anesthetic agent administered to the patient via the inspiratory port 218.

Further, the adjustments described above may be facilitated at least in part based on output from the respiratory gas module 208. The respiratory gas module 208 may be configured to measure various parameters of the gases exiting the vaporizer and/or being provided to the patient. For example, the respiratory gas module 208 may measure the concentrations of carbon dioxide, nitrous oxide, and the anesthetic agent provided to the patient. Further, the respiratory gas module 208 may measure respiration rate, minimum alveolar concentration, patient oxygen, and/or other parameters. The output from the respiratory gas module 208 may be displayed via a graphical user interface on a display device (e.g., the anesthesia display device 204 and/or the patient monitoring display device 206) and/or used by a controller to provide closed-loop feedback control of the amount of anesthesia provided to the patient.

The ventilator 212 may optionally be coupled to a breathing circuit (not shown) including a plurality of tubes (e.g., gas passages) 222. The breathing circuit may be coupled between an airway of a patient (e.g., via a breathing mask positioned to enclose the mouth and/or nose of the patient or a tracheal intubation tube) and the inspiratory port 218. Gases (e.g., the one or more medical gases, or a mixture of the one or more medical gases and vaporized anesthetic agent from the anesthetic vaporizer 214) may flow from the inspiratory port 218, through the breathing circuit, and into the airway of the patient, where the gases are absorbed by the lungs of the patient. By adjusting the concentration of vaporized anesthetic agent in the gases as described above, the operator may adjust a degree to which the patient is anesthetized.

During conditions in which the breathing circuit is coupled to the airway, the anesthetic agent and/or fresh gas (without the anesthetic agent) may flow into the airway of the patient (e.g., through inhalation) via the inspiratory port 218 and the inspiratory check valve. As an example, the inspiratory check valve may open automatically (e.g., without input or adjustment by the operator) in response to inhalation by the patient and may close automatically in response to exhalation by the patient. Similarly, the expiratory check valve may open automatically in response to exhalation by the patient and may close automatically in response to inhalation by the patient.

In some embodiments, the operator may additionally or alternatively control one or more operating parameters of the anesthesia machine 200 via an electronic controller 240 of the anesthesia machine 200. Controller 240 includes a processor operatively connected to a memory. The memory may be a non-transitory computer-readable medium and may be configured to store computer executable code (e.g., instructions) to be processed by the processor in order to execute one or more routines, such as those described herein. The memory may also be configured to store data received by the processor. Controller 240 may be communicatively coupled (e.g., via wired or wireless connections) to one or more external or remote computing devices, such as a hospital computing system, and may be configured to send and receive various information, such as electronic medical record information, procedure information, and so forth. Controller 240 may also be electronically coupled to various other components of the anesthesia machine 200, such as the anesthetic vaporizer 214, the ventilator 212, the respiratory gas module 208, the anesthesia display device 204, and the patient monitoring display device 206.

The controller 240 receives signals from the various sensors of the anesthesia machine 200 and employs the various actuators of the anesthesia machine 200 to adjust operation of the anesthesia machine 200 based on the received signals and instructions stored on the memory of the controller. For example, the flow of gases to the inspiratory port 218 may be controlled via an input device (e.g., keyboard, touchscreen, etc.) coupled to the electronic controller of the anesthesia machine 200. The controller 240 may display operating parameters of the anesthesia machine 200 via the anesthesia display device 204 and/or the patient monitoring display device 206. The controller may receive signals (e.g., electrical signals) via the input device and may adjust operating parameters of the anesthesia machine 200 in response (e.g., responsive) to the received signals.

As one example, the operator may input a desired concentration of the anesthetic agent to be delivered to the patient. A corresponding valve position of one or more valves of the anesthesia machine (e.g., a position of one or more bypass valves, as described above) may be empirically determined and stored in a predetermined lookup table or function in a memory of the controller. For example, the controller may receive the desired concentration of the anesthetic agent via the input device and may determine an amount of opening of the one or more valves corresponding to the desired concentration of the anesthetic agent based on the lookup table, with the input being the concentration of the anesthetic agent and the output being the valve position of the one or more valves. The controller may transmit an electrical signal to an actuator of the one or more valves in order to adjust each of the one or more valves to the corresponding output valve position. In some examples, the controller may compare the desired flow rate of gases to a measured flow rate of gases, such as measured by the inspiratory flow sensor, for example.

Controller 240 is shown in FIG. 2 for illustrative purposes, and it is to be understood that controller 240 may be located in various locations within, around, and/or remote from anesthesia machine 200. As an example, controller 240 may include multiple devices/modules that may be distributed throughout anesthesia machine 200. As such, controller 240 may include a plurality of controllers at various locations within anesthesia machine 200. As another example, additionally or alternatively, controller 240 may include one or more devices/modules that are external to anesthesia machine 200, located proximate to (e.g., in a same room) or remote from (e.g., a remote server) anesthesia machine 200. In each example, the multiple devices/modules may be communicatively coupled through wired and/or wireless connections.

Anesthetic vaporizers, such as anesthetic vaporizer 214 shown in FIG. 2, may employ various methods to vaporize a liquid anesthetic agent. For example, the anesthetic vaporizer may use a flow-over method (in which a carrier gas flows over a top surface of a volatile liquid anesthetic agent), a bubble-through method (in which the carrier gas is bubbled up through the liquid anesthetic agent), or a gas/vapor blender (in which heat is used to vaporize the liquid anesthetic agent, and the vapors are injected into a fresh gas flow). Regardless of the vaporization method, in some embodiments, the anesthetic vaporizer 214 may include a refillable sump for storing the liquid anesthetic agent before it is delivered to a vaporizing chamber.

Referring to FIG. 3, an example ventilator assembly 300 is shown schematically. The ventilator assembly 300 may be similar to, or the same as, the ventilator 112 shown by FIG. 1 and described above and/or the ventilator 212 shown by FIG. 2 and described above. For example, the ventilator assembly 300 may be included within the anesthesia machine 199 shown by FIG. 1 and/or the anesthesia machine 200 shown by FIG. 2.

The ventilator assembly 300 includes a drive gas unit 326. The drive gas unit 326 may receive ventilator drive gas via a ventilator drive gas source 333. The ventilator drive gas source 333 may be a gas pipeline in some embodiments, and in other embodiments the ventilator drive gas source 333 may be a gas cylinder. A composition of the ventilator drive gas provided by the ventilator drive gas source 333 may be based on an intended use and/or intended use environment of the anesthesia machine including the ventilator assembly 300. For example, in some anesthesia machines, the composition of the ventilator drive gas may be 100 percent oxygen, and in other anesthesia machines the ventilator drive gas may be 100 percent air. Other examples are possible. In some configurations, the ventilator drive gas source 333 may be hard-plumbed to the anesthesia machine via a pipeline or other apparatus. In other configurations, the ventilator drive gas source 333 may be a gas cylinder that may be coupled or decoupled from the anesthesia machine for replacement, refilling, maintenance, etc.

The ventilator assembly 300 includes a plurality of valves and other devices of ventilator drive gas circuit 352 that may be controlled by the electronic controller of the anesthesia machine in order to adjust operation of the ventilator assembly 300. For example, the controller of the anesthesia machine, which may be similar to, or the same as, the controller 162 described above with reference to FIG. 1 and/or the controller 240 described above with reference to FIG. 2, may adjust operation of valve 302 of the drive gas unit 326 in order to control the flow of ventilator drive gas from the ventilator drive gas source 333. As one example, the controller may communicate with the valve 302 by transmitting signals (e.g., electronic signals) to an actuator of the valve 302 to command the valve to a fully opened position, a fully closed position, or a plurality of different positions between the fully opened position and the fully closed position. In some examples, the valve 302 may be a solenoid valve adjustable from the fully closed position to the fully opened position or vice versa. The controller of the anesthesia machine may also communicate with other valves and components of the ventilator assembly 300, such as valve 330, valve 324, valve 334 and valve 336 (where valve 334 and valve 336 may be included by multi-gas unit 328 which may be included by the ventilator assembly 300, as described below), etc. In some examples, valve 330 may be controlled directly (e.g., by hand) by a user of the anesthesia machine (e.g., a clinician) to turn off the flow of ventilator drive gas from ventilator drive gas source 333.

The ventilator assembly 300 may include a plurality of gas filters configured to reduce a likelihood of introduction of impurities into the gas passages and other components of the ventilator assembly 300. For example, in the embodiment shown, the ventilator assembly 300 includes filter 332, filter 316, and filter 320. In configurations in which the ventilator assembly 300 includes the multi-gas unit 328, the ventilator assembly 300 may further include filter 335 and filter 338. The ventilator assembly 300 may further include drive gas flow sensor 304, drive gas pressure sensor 314, airway pressure sensor 318, switch 344, exhalation valve 346, and expiratory flow sensor 348. The drive gas flow sensor 304 may provide feedback for the inspiratory flow valve 302 (e.g., communicate electronically with the controller and/or inspiratory flow valve 302), the drive gas pressure sensor 314 may monitor (e.g., sense) a pressure of gases within the drive gas circuit, the airway pressure sensor 318 may monitor a pressure of gases within the inspiratory circuit, switch 344 may adjust operation of bag 603, exhalation valve 346 may adjust the anesthesia machine between an inspiration mode (e.g., with exhalation valve 346 closed) or an expiration mode (e.g., with exhalation valve 346 open), and expiratory flow sensor 348 may monitor (e.g., sense) expiratory flow with the controller calculating expiratory volumes based on an output of the expiratory flow sensor 348.

Ventilator drive gas may flow from the ventilator drive gas source 333 to one or both of drive regulator 312 and pilot regulator 322. Gas flowing through the drive regulator 312 may flow to connection 350 of the ventilator assembly 300, which may fluidly connect the ventilator drive gas source 333 to an inspiratory circuit of the anesthesia machine. An example inspiratory circuit which may be included within the anesthesia machine is described further below with reference to FIG. 5.

In some configurations, the ventilator assembly 300 may include multi-gas unit 328, and the multi-gas unit 328 may provide ventilator drive gases to the passages of the ventilator assembly 300 from different ventilator drive gas sources. In particular, the multi-gas unit 328 may be configured with at least two ventilator drive gas sources, and the controller of the anesthesia machine may communicate with the multi-gas unit 328 (e.g., transmit electronic signals to valves of the multi-gas unit 328) in order to adjust a selection of which ventilator drive gas source provides ventilator drive gas to the passages of the ventilator assembly 300. For example, during some conditions, the controller of the anesthesia machine may communicate with the valve 334 in order to adjust the valve 334 to the fully closed position, and the controller may further communicate with the valve 336 in order to adjust the valve 336 to the fully opened position. In this configuration, ventilator drive gas may be provided to the passages and other components of the ventilator assembly (e.g., the drive regulator 312) from a first ventilator drive gas source 339 of the multi-gas unit 328, while gas may not flow from a second ventilator drive gas source 341 of the multi-gas unit 328. Responsive to a user input to a user interface device provided by a user of the anesthesia machine (e.g., a clinician), the controller may command a switching of the selected ventilator drive gas source from the first ventilator drive gas source 339 of the multi-gas unit 328 to the second ventilator drive gas source 341 of the multi-gas unit 328. Switching the selected ventilator drive gas source may include communicating with the valve 336 in order to adjust the valve 336 to the fully closed position, and communicating with the valve 334 in order to adjust the valve 334 to the fully opened position. As a result, ventilator drive gas may be provided to the passages and other components of the ventilator assembly from the second ventilator drive gas source 341 of the multi-gas unit 328, while gas may not flow from the first ventilator drive gas source 339 of the multi-gas unit 328. In this way, operation of the ventilator assembly 300 may be adjusted from flowing ventilator drive gas provided by the first ventilator drive gas source 339 to the inspiratory circuit, to flowing ventilator drive gas provided by the second ventilator drive gas source 341 to the inspiratory circuit.

Referring to FIG. 4, an example gas mixer 400 is shown schematically. The gas mixer 400 may be similar to, or the same as, the gas mixer 163 described above with reference to FIG. 1 and/or the gas mixer 217 described above with reference to FIG. 2. The gas mixer 400 is configured to be included within an anesthesia machine, such as the anesthesia machine 199 shown by FIG. 1 and described above, or the anesthesia machine 200 shown by FIG. 2 and described above.

The gas mixer 400 is configured to flow gases from one or more gas mixer sources (which may be referred to herein as mixer gas sources) to an inspiratory port of the anesthesia machine for inhalation of the gases by a patient. In the embodiment shown, the gas mixer 400 is configured to receive gases from a first gas mixer source 452 via connection 453, a second gas mixer source 454 via connection 455, and/or a third gas mixer source 456 via connection 457. Gas mixer 400 may receive the gases from one or more of the gas mixer sources and may combine (e.g., mix) the gases from the one or more gas mixer sources to form a mixture of gases for delivery to the inspiratory port of the anesthesia machine. In some embodiments, the mixture of gases may flow from the gas mixer 400 through a vaporizer, such as the vaporizer 114 described above with reference FIG. 1 or the vaporizer 214 described above with reference to FIG. 2, where the mixture of gases may combine with vaporized anesthetic agents prior to flowing to the inspiratory port.

Each of the gas mixer sources may be configured to include a different type of gas (e.g., a gas having a different composition relative to gases of each other gas mixer source). As one example, the first gas mixer source 452 may provide 100 percent oxygen to the gas mixer 400, the second gas mixer source 454 may provide 100 percent pure air to the gas mixer 400, and the third gas mixer source 456 may provide 100 percent nitrous oxide to the gas mixer 400. However, other gas compositions and/or combinations are possible.

The gas mixer 400 may include a plurality of valves that may be controlled by the electronic controller of the anesthesia machine in order to adjust operation of the gas mixer 400. For example, gas mixer 400 includes valve 408, valve 410, valve 414, valve 430, valve 436, and valve 448. The gas mixer 400 may further include a plurality of gas filters configured to reduce a likelihood of introduction of gas impurities to other components of the anesthesia machine. For example, the gas mixer 400 is shown including filter 402, filter 404, filter 406, filter 418, filter 422, filter 428, filter 434, filter 440, filter 444, and filter 446. The gas mixer 400 may further include check valve 412, check valve 424, check valve 432, and check valve 450. Gases flowing across flow sensor 420 and flow sensor 442 may be mixed together (e.g., combined) to form a gas mixture which may flow to the inspiratory port of the anesthesia machine. Pressure sensor 416 may measure a pressure of gases flowing to flow sensor 420, pressure sensor 438 may measure a pressure of gases flowing to flow sensor 442, and pressure sensor 426 may measure a pressure of gases downstream of each of flow sensor 420 and flow sensor 442.

Referring to FIG. 5, an example inspiratory circuit 500 including a bellowless plenum 502 of an anesthesia machine is shown. The inspiratory circuit 500 and bellowless plenum 502 may be included within the anesthesia machine 199 described above with reference to FIG. 1 and/or the anesthesia machine 200 described above with reference to FIG. 2. The inspiratory circuits described herein, such as inspiratory circuit 500, may be referred to as bellowless breathing systems.

Passages of the inspiratory circuit 500 may receive gases from a ventilator and/or gas mixer and may direct the flow of the gases to an inspiratory port 518 (which may be referred to herein as a patient inspiratory port) for inhalation of the gases by a patient. The ventilator may be similar to, or the same as, the ventilator 112 described above with reference to FIG. 1, ventilator 212 described above with reference to FIG. 2, and/or ventilator assembly 300 described above with reference to FIG. 3. The gas mixer may be similar to, or the same as, the gas mixer 163 described above with reference to FIG. 1, the gas mixer 217 described above with reference to FIG. 2, and/or the gas mixer 400 described above with reference to FIG. 4. The inspiratory port may be similar to, or the same as, the inspiratory port 218 described above with reference to FIG. 2.

The inspiratory circuit 500 includes a connection 516 fluidly coupling an inlet 524 (which may be referred to herein as a first port) of the bellowless plenum 502 to a ventilator drive gas source. During conditions in which a user (e.g., clinician) inputs a selection via a user interface device to operate the anesthesia machine in a high flow mode, the ventilator may flow ventilator drive gases from the ventilator drive gas source to the bellowless plenum 502. While the ventilator drive gases flow to the bellowless plenum 502, the anesthesia machine may maintain an exhalation valve of the anesthesia machine in a fully closed position The ventilator drive gases may flow through one or more passages 504 in a serpentine arrangement within the bellowless plenum 502 to an outlet 526 (which may be referred to herein as a second port) of the bellowless plenum 502 and may flow from the outlet 526 to the inspiratory port 518. The ventilator drive gases provided to the inspiratory port 518 may flow through a nasal cannula for inhalation by a patient, in some embodiments.

The inspiratory circuit 500 includes a plurality of valves and other components that may control a flow of gases through the inspiratory circuit 500. For example, in the embodiment shown by FIG. 5, the inspiratory circuit 500 includes valve 506, valve 508, check valve 514, and check valve 510. In some examples, an electronic controller of the anesthesia machine (e.g., electronic controller 162 shown by FIG. 1 and described above, electronic controller 240 shown by FIG. 2 and described above, etc.) may transmit signals (e.g., electronic signals) to actuators of the valves to adjust one or more of the valves from a fully opened position to a fully closed position, or vice versa, to control the flow direction of ventilator drive gases through the inspiratory circuit 500. In other examples, one or more of the valves may be mechanical valves configured to operate without actuation (e.g., check valve 514 and check valve 510 may be mechanical valves configured to flow gases in a first direction and to reduce a likelihood of flowing the gases in an opposite, second direction).

During some conditions, gases from the gas mixer may be provided to the passages of the inspiratory circuit 500 via connection 522. For example, during conditions in which the high flow mode of the anesthesia machine is not selected, gases from the gas mixer may flow to the inspiratory circuit 500 and ventilator drive gases from the ventilator drive gas source may not flow through the bellowless plenum 502 to the outlet 526 of the bellowless plenum 502. As another example, during conditions in which the high flow mode of the anesthesia machine is selected and the controller determines that user-selected parameters of gas to be delivered to the inspiratory port may be fulfilled by combining ventilator drive gas with gas from the gas mixer, the controller may adjust operation of the anesthesia machine to flow ventilator drive gas through the bellowless plenum and combine the ventilator drive gas with gas from the gas mixer at a location downstream of the outlet of the bellowless plenum. The combined gas (e.g., gas mixture) may then flow to the inspiratory port for inhalation by the patient.

During conditions in which gases exhaled by the patient are returned to the anesthesia machine via connection 520 (e.g., conditions in which ventilator drive gas is not provided to the inspiratory port), absorber 512 may remove carbon dioxide from the exhaled gases and may flow the exhaled gases with the carbon dioxide removed to the inspiratory port for re-inhalation by the patient.

Referring to FIG. 6, an anesthesia machine 600 is schematically shown with an example arrangement of components according to an embodiment. In particular, the anesthesia machine 600 includes the gas mixer 400 shown by FIG. 4 and described above, the inspiratory circuit 500 shown by FIG. 5 and described above, and the ventilator assembly 300 shown by FIG. 3 and described above.

Flow pathway 602 included by FIG. 6 illustrates a flow path of gases through the gas mixer 400 and other components of the anesthesia machine 600 toward the inspiratory port 518 of the inspiratory circuit 500. In the example shown, gases flow to the gas mixer 400 via connection 453 fluidly coupled to the first gas mixer source 452 (shown by FIG. 4) and connection 455 fluidly coupled to the second gas mixer source 454 (shown by FIG. 4), and the mixer gases mixed by the gas mixer 400 flow through vaporizer 612 where the gases may combine with vaporized anesthetic agent. The vaporizer 612 may be similar to, or the same as, the vaporizer 114 shown by FIG. 1 and described above and/or the vaporizer 214 shown by FIG. 2 and described above. The gases may flow from the vaporizer to the inspiratory port 518 for inhalation by the patient.

Further, FIG. 6 shows flow pathway 604 illustrating a flow path of ventilator drive gases from the ventilator drive gas source 333 through the bellowless plenum 502 to the inspiratory port 518. The ventilator drive gases may mix (e.g., combine) with gases from the gas mixer 400 within a passage 614 downstream of the bellowless plenum 502 to form a gas mixture for inhalation by the patient. In the embodiment shown, the anesthesia machine 600 includes a nasal cannula 608 that may receive the gases from the gas mixer 400, the ventilator drive gases from the ventilator drive gas source 333, and/or the mixture of the gases from the mixer 400 and the ventilator drive gases via the inspiratory port 518. During conditions in which the ventilator drive gases flow to the inspiratory port 518, the electronic controller of the anesthesia machine 600 may maintain exhalation valve 616 in a fully closed position such that ventilator drive gas flowing along flow pathway 604 does not flow to exhalation flow pathway 610.

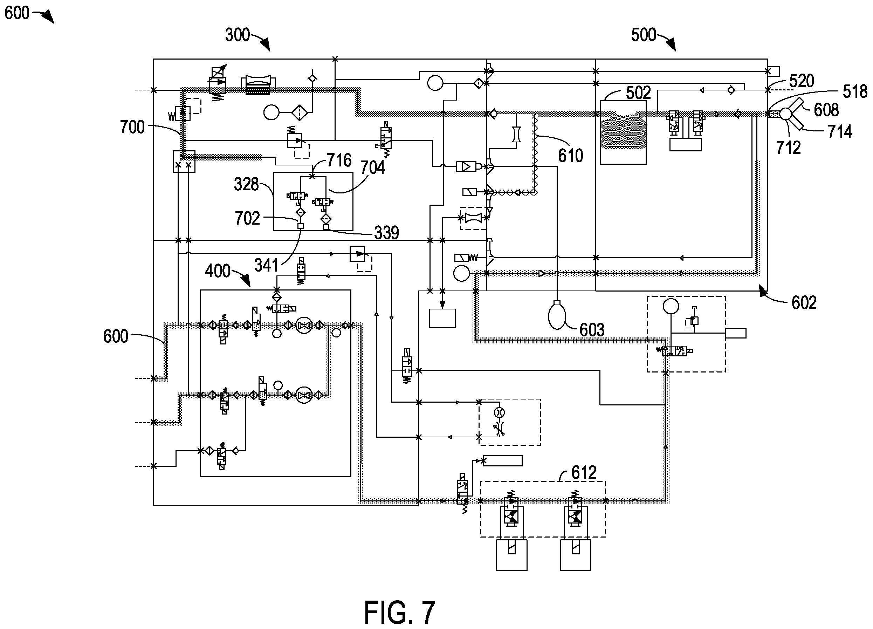

Referring to FIG. 7, anesthesia machine 600 is shown schematically and is configured to include the multi-gas unit 328 described above with reference to FIG. 3. FIG. 7 further shows ventilator drive gas flowpaths from the multi-gas unit 328. In particular, FIG. 7 shows ventilator drive gas flowpath 700 between an outlet 716 of the multi-gas unit 328 and inspiratory port 518. Ventilator drive gases may flow from the multi-gas unit 328 through the bellowless plenum 502 to the inspiratory port 518.

The electronic controller of the anesthesia machine 600 may communicate electronically with the multi-gas unit 328 in order to adjust the ventilator drive gas source providing ventilator drive gas along the ventilator drive gas flowpath 700. As example, the electronic controller may adjust the valves of the multi-gas unit 328 as described above with reference to FIG. 3 to flow ventilator drive gas from the first ventilator drive gas source 339 to the inspiratory port 518 along gas pathway 704 and ventilator drive gas flowpath 700. As another example, the electronic controller may adjust the valves of the multi-gas unit 328 as described above with reference to FIG. 3 to flow ventilator drive gas from the second ventilator drive gas source 341 to the inspiratory port 518 along gas pathway 702 and ventilator drive gas flowpath 700. The composition of gas provided by the first ventilator drive gas source 339 may be different than the composition of gas provided by the second ventilator drive gas source 341 such that adjusting the composition of the ventilator drive gas flowing to the inspiratory port 518 along ventilator drive gas flowpath 700 may be controlled by the electronic controller responsive to user input (e.g., user selection) provided to the anesthesia machine 600 via a user interface device (e.g., a keyboard, touchscreen, trackball, etc.). In one example, the gas provided by the first ventilator drive gas source 339 may be pure oxygen and the gas provided by the second ventilator drive gas source 341 may be a mixture of oxygen and air. However, other gas compositions are possible.

In the embodiment shown by FIG. 7, the anesthesia machine 600 includes a gas diverter 712 (which may be referred to herein as a multi-outlet gas diverter) coupled to the inspiratory port 518. The gas diverter 712 may be adjusted by the user (e.g., clinician) in order to direct the flow of gas from the inspiratory port 518 to the nasal cannula 608 or to a diverter inspiratory port 714 of the gas diverter 712. By configuring the anesthesia machine 600 to include the gas diverter 712, a high flow of ventilator drive gas provided to the inspiratory port 518 may be directed to either the nasal cannula 608 or the diverter inspiratory port 714 based on a clinical operation being performed and without decoupling the nasal cannula 608 from the anesthesia machine 600. For example, the user may adjust the gas diverter 712 to flow ventilator drive gases through the gas diverter 712 to the nasal cannula 608, or the user may adjust the gas diverter 712 to flow gases (e.g., mixer gases) through the gas diverter 712 to the diverter inspiratory port 714 (e.g., for normal ventilation of the patient).

Referring to FIG. 8, a flowchart illustrating a method for controlling gas flow in an anesthesia machine is shown. The anesthesia machine may be similar to, or the same as, the anesthesia machine 199 shown by FIG. 1 and described above, anesthesia machine 200 shown by FIG. 2 and described above, and/or anesthesia machine 600 shown by FIGS. 6-7 and described above. The method may be performed by an electronic controller of the anesthesia machine based on instructions stored in non-transitory memory of the controller and in combination with various actuators of the anesthesia machine. For example, the electronic controller may be similar to, or the same as, the electronic controller 162 shown by FIG. 1 and described above, electronic controller 240 shown by FIG. 2 and described above, and/or the electronic controller of anesthesia machine 600 described above.

At operation 802, the method includes estimating and/or measuring anesthesia machine operating conditions. Estimating and/or measuring the anesthesia machine operating conditions may include estimating and/or measuring a flow rate of gas from a gas mixer of the anesthesia machine and/or a ventilator drive gas source of the anesthesia machine, a selection of a ventilator drive gas source (e.g., for configurations of a ventilator assembly of the anesthesia machine including a multi-gas unit), a rate of vaporization of anesthetic agent within a vaporizer of the anesthesia machine, a condition of valves of the anesthesia machine, etc.

The method continues from operation 802 to operation 804 where the method includes determining whether a high flow gas mode has been selected. The high flow gas mode of the anesthesia machine may be a mode selected by a user of the anesthesia machine (e.g., a clinician). During conditions in which the high flow gas mode is selected, the electronic controller of the anesthesia machine may control the operation of the ventilator assembly to flow ventilator drive gas from a ventilator drive gas source through a bellowless plenum separating the ventilator drive gas source from a mixer gas source as described further below. The ventilator drive gas may flow through the bellowless plenum to an inspiratory port of the anesthesia machine for inhalation by a patient. As one example, the inspiratory port may be coupled to a nasal cannula, and the ventilator drive gas may flow through the inspiratory port into the nasal cannula for inhalation by the patient. The high gas flow mode may increase an ease of performing clinical operation such as intubation of the patient by flowing ventilator drive gas to the patient at a high flow rate, which may provide an increased amount of time for the intubation to be performed.

If a high flow gas mode has not been selected at operation 804, the method continues from operation 804 to operation 806 where the method includes maintaining anesthesia machine operating conditions. Maintaining the anesthesia machine operating conditions may include maintaining the flow rate of gases from the anesthesia machine, maintaining the position (e.g., amount of opening) of valves of the anesthesia machine, maintaining the selection of the ventilator drive gas source, etc.

However, if a high flow gas mode has been selected at operation 804, the method continues from operation 804 to operation 807 where the method includes stopping a flow of anesthetic agent from a vaporizer of the anesthesia machine throughout an entire duration of the high gas flow mode operation. Stopping the flow of anesthetic agent from the vaporizer may include diverting (e.g., bypassing) the flow of gas from the gas mixer around the vaporizer such that gases flowing from the gas mixer toward an inspiratory port of the anesthesia machine do not flow through the vaporizer and the gases are not mixed with anesthetic agent within the vaporizer. As another example, stopping the flow of anesthetic agent from the vaporizer may include adjusting the vaporizer to an OFF condition, such that flowing gases from the gas mixer through the vaporizer does not mix the gases flowing from the gas mixer with anesthetic agent (e.g., the vaporizer be de-energized and may not vaporize anesthetic agent while maintained in the OFF condition).

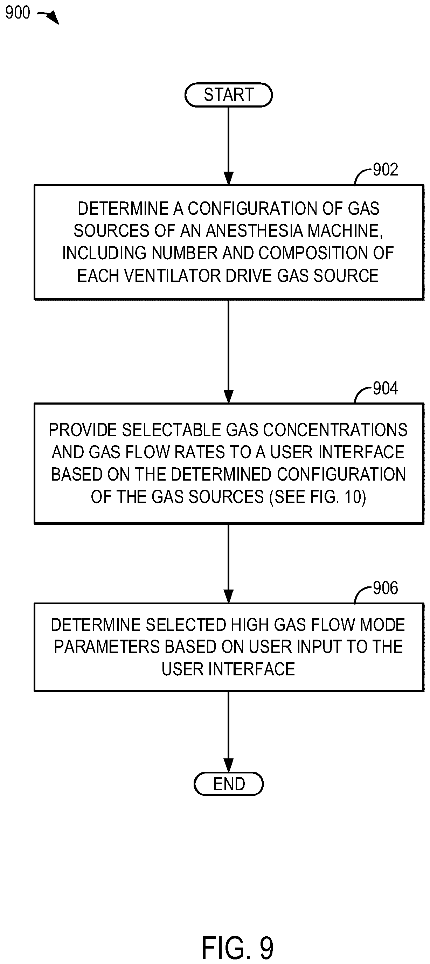

The method continues from operation 807 to operation 808 where the method includes determining an amount of ventilator drive gas to be delivered to the inspiratory port based on selected high gas flow mode parameters. Determining the amount of ventilator drive gas to be delivered to the inspiratory port may include determining a flow rate of the ventilator drive gas from the ventilator drive gas source to the bellowless plenum of the anesthesia machine. Determining the amount of ventilator drive gas to be delivered to the inspiratory port may further include determining a concentration of the ventilator drive gas to be delivered to the inspiratory port (e.g., a gas ratio of ventilator drive gas to mixer gas). The high gas flow mode parameters such as ventilator drive gas flow rate and ventilator drive gas concentration may be selected by the user of the anesthesia machine and may be based on a configuration of gas sources of the anesthesia machine, as described further below with reference to FIG. 9. According to an embodiment, operations 808 may be performed before operation 807 or the operations may be performed simultaneously.

The method continues from operation 808 to operation 810 where the method includes flowing ventilator drive gas from a ventilator drive gas source through the bellowless plenum, which separates a drive gas circuit of the ventilator from an inspiratory gas circuit including the inspiratory port. The bellowless plenum may be similar to, or the same as, the bellowless plenum 133 described above with reference to FIG. 1, the bellowless plenum 219 described above with reference to FIG. 2, and/or the bellowless plenum 502 described above with reference to FIGS. 5-7. The drive gas circuit may be similar to, or the same as, the ventilator drive gas circuit 352 shown by FIG. 3, and the inspiratory gas circuit may be similar to, or the same as, the inspiratory circuit 500 shown by FIG. 5 and described above. Flowing the ventilator drive gas through the bellowless plenum may include flowing the ventilator drive gas to the inlet of the bellowless plenum, flowing the ventilator drive gas from the inlet through one or more serpentine passages within the bellowless plenum, and flowing the ventilator drive gas from the serpentine passages out of an outlet of the bellowless plenum. The inlet and outlet of the bellowless plenum may be similar to, or the same as, the inlet 524 and the outlet 526, respectively, described above with reference to FIG. 5. While the ventilator drive gas flows through the bellowless plenum, the electronic controller may maintain an exhalation valve of the anesthesia machine in a fully closed position, similar to the examples described above. The exhalation valve may be similar to, or the same as, the exhalation valve 616 shown by FIGS. 6-7 and described above.

The method continues from operation 810 to operation 812 where the method includes delivering the ventilator drive gas flowing through the bellowless plenum to the inspiratory port. The ventilator drive gas may flow from the outlet of the bellowless plenum to the inspiratory port. As one example, the flow path of the ventilator drive gas from the bellowless plenum to the inspiratory port may be the same as the flow path 604 shown by FIG. 6 and described above. While flowing the ventilator drive gas from the ventilator drive gas source through the bellowless plenum to the inspiratory port, an entire amount of the ventilator drive gas output by the ventilator drive gas source may flow through the bellowless plenum to the inspiratory port (e.g., the flow rate of ventilator drive gas from the ventilator drive gas source may be equal to the flow rate of ventilator drive gas to the inspiratory port), in some examples.

In some examples, the ventilator drive gas may be provided to the inspiratory port for inhalation by a patient without mixing the ventilator drive gas with other gases. For example, the ventilator drive gas may be pure air and may be inhaled by the patient for patient respiration. In other examples, the ventilator drive gas may be mixed with other gases prior to delivery to the inspiratory port in order to provide a user-selected concentration and flow rate of gases to the inspiratory port for inhalation by the patient, as described below.

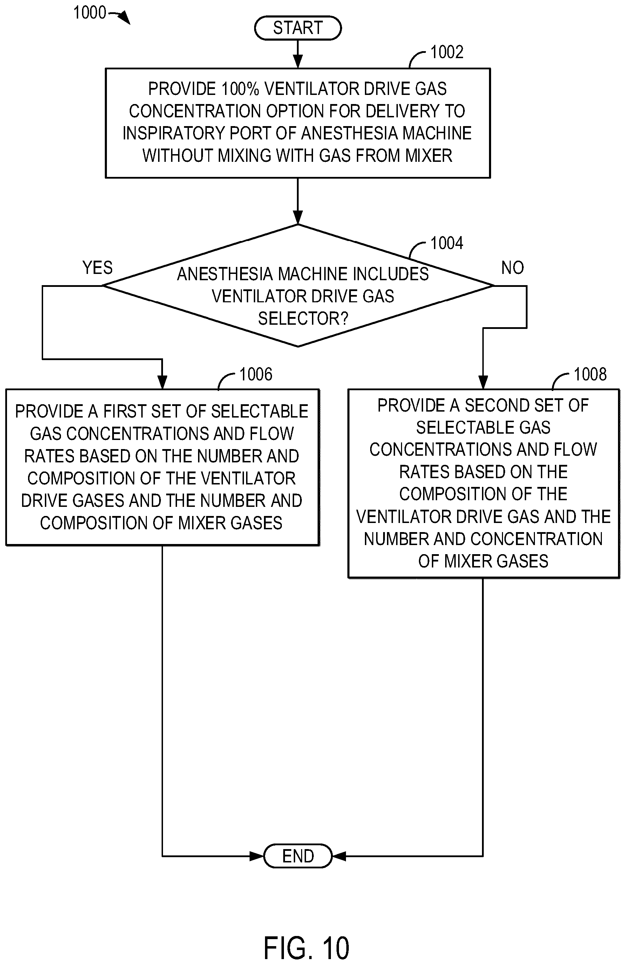

The method at operation 812 may include, at operation 814, mixing the ventilator drive gas with gas provided by a mixer of the anesthesia machine, and delivering the mixture of the ventilator drive gas and the mixer gas to the inspiratory port. In some examples, the gas provided by the mixer of the anesthesia machine may have a different composition relative to the composition of the ventilator drive gas, and by mixing the gas provided by the mixer with the ventilator drive gas, various gas mixtures may be provided. For example, the ventilator drive gas may be 100 percent oxygen, and the gas provided by the mixer of the anesthesia machine may be 100 percent air. By adjusting the flow rate of the ventilator drive gas and adjusting the flow rate of the gas provided by the mixer of the anesthesia machine, different ratios of ventilator drive gas to mixer gas may be achieved, which may result in different concentrations of oxygen of the mixture of the ventilator drive gas and the mixer gas. Various examples are described below with reference to FIG. 10.

The method continues from operation 812 to operation 816 where the method includes flowing the ventilator drive gas through the inspiratory port to a nasal cannula. The nasal cannula may be coupled to the patient (e.g., provided at a nose of the patient) and the gases provided to the inspiratory port, including the ventilator drive gas, may flow through the nasal cannula for inhalation by the patient. By providing the ventilator drive gases to the inspiratory port for inhalation by the patient via the nasal cannula, the gases may be provided to the patient with a higher flow rate than gases flowing only from the gas mixer. As one example, the ventilator drive gas may flow at rates within a range of 45 to 60 liters per minute, and the gases flowing from the gas mixer may flow at rates within a range of 0 to 15 liters per minute. The ventilator drive gas may therefore provide an increased amount of gases such as air, oxygen, etc. to the patient for various clinical operations such as patient intubation. Because the anesthesia machine is configured to include the bellowless plenum, electronic ventilator, and electronic gas mixer, the flow of gas through the anesthesia machine may be more precisely controlled, which may provide for more precise calibration of a gas ratio of gas provided to the inspiratory port. The ventilator drive gas may be driven through the bellowless plenum to the inspiratory port, whereas configurations that include a bellows unit separating the ventilator from the inspiratory port may not provide a pathway for ventilator drive gas to travel to the inspiratory port. For example, in configurations including a bellows unit, the ventilator drive gas may flow against external surfaces of the bellows in order to drive compression of the bellows but may not flow downstream of the bellows to the inspiratory port (e.g., the bellows may act as a physical barrier separating the flow of ventilator drive gases from the flow of gases from the gas mixer). Thus, the flow is limited to the 0 to 15 liters per minute that is provided by the gas mixer, which may be an insufficient flow rate for use with a high flow nasal cannula.

Referring to FIG. 9, a flowchart illustrating a method 900 for selecting high gas flow mode parameters of an anesthesia machine is shown. The anesthesia machine may be a same anesthesia machine described above with reference to FIG. 8, and the method 900 may be executed as part of method 800 described above. Components described herein with reference to method 900 may be the same as those described above with reference to method 800 and may not be re-introduced.

The method at operation 902 includes determining a configuration of gas sources of an anesthesia system, including a number and a composition of each ventilator drive gas source. Determining the configuration of the gas sources may include determining a number of mixer gas sources and composition of the mixer gas sources of the anesthesia machine. For example, the gas mixer of the anesthesia machine may be configured to receive gases from at least one gas mixer source, and in some examples the gas mixer may receive gases from multiple gas mixer sources and may mix the gases from the gas mixer sources prior to flowing the gases toward the inspiratory port of the anesthesia machine. The gas mixer sources may be similar to, or the same as, the gas mixer sources described above. As one example, the gas mixer may be coupled to a first gas mixer source, a second gas mixer source, and a third gas mixer source, such as the first gas mixer source 452, the second gas mixer source 454, and third gas mixer source 456, respectively, described above with reference FIG. 4. The controller may determine the number and composition of the gas mixer sources via user input to the anesthesia machine via a user interface device, in some examples. In other examples, the controller may sense the number and composition of the gas mixer sources electronically (e.g., via radio frequency identification, or RFID, tags coupled to each gas mixer source, or by detection of another identification feature of the gas sources). In yet other examples, the controller may determine the number and composition of the gas mixer sources by measuring the pressure of the gas mixer sources (e.g., gas from a first gas mixer source may be provided to the gas mixer at a first pressure and gas from a second gas mixer source may be provided to the gas mixer at a different, second pressure, with the controller identifying the gases based on the first pressure and the second pressure).

The electronic controller may similarly determine the number and composition of ventilator drive gas sources included by the anesthesia machine. In some examples, the anesthesia machine may include a single ventilator drive gas source, such as a hard-plumbed ventilator drive gas pipeline coupled to the anesthesia machine, or a single ventilator drive gas cylinder coupled to the anesthesia machine. In other examples, the anesthesia machine may include multiple ventilator drive gas sources, and the electronic controller may control which ventilator drive gas source provides ventilator drive gas to the ventilator assembly and inspiratory port of the anesthesia machine. For example, the anesthesia machine may include a multi-gas unit having a plurality of different ventilator drive gas sources, such as the multi-gas unit 328 described above with reference to FIG. 3. The electronic controller may adjust a position (e.g. amount of opening) of the various valves of the ventilator assembly in order to provide a selected ventilator drive gas to the passages of the ventilator assembly. In some examples, the electronic controller may determine the number and composition of the ventilator drive gas sources based on user input provided via the user interface device, and in other examples the electronic controller may determine the number and composition of the ventilator drive gas sources based on identification features of the ventilator sources (e.g., RFID tags as described above) and/or pre-determined lookup tables stored in non-transitory memory of the electronic controller provided during manufacturing of the anesthesia machine.

The method continues from operation 902 to operation 904 where the method includes providing selectable gas concentrations and gas flow rates to a user interface based on the determined configuration of the gas sources. The selectable gas concentrations and gas flow rates may be determined by the electronic controller based on the number and composition of the gas sources of the anesthesia machine. In particular, the electronic controller may determine the selectable gas concentrations and gas flow rates based on the number and composition of ventilator drive gas sources and the number and composition of mixer gas sources. Various examples are described further below with reference to FIG. 10.

The method continues from operation 904 to operation 906 where the method includes determining selected high gas flow mode parameters based on a user input to the user interface. The user (e.g. clinician) may input a selection of parameters of the high gas flow mode to be provided by the anesthesia machine using the determined selectable gas concentrations and gas flow rates provided by the controller. In particular, the electronic controller may provide a plurality of different selectable gas concentrations and gas flow rates, and the user may select the desired gas flow rate and gas concentration from the available options. Responsive to selection of the parameters of the high gas flow mode by the user, the electronic controller may adjust operation of the anesthesia machine to flow ventilator drive gas and/or a mixture of ventilator drive gas and gas from the gas mixer to the inspiratory port of the anesthesia machine, as described above with reference to FIG. 8. The gases flowing to the inspiratory port have the selected flow rate and composition (e.g., concentration of oxygen, air, etc.) input by the user.

Referring to FIG. 10, a flowchart illustrating a method 1000 for selecting high gas flow mode parameters of an anesthesia machine is shown. The anesthesia machine may be a same anesthesia machine described above with reference to FIGS. 8-9, and the method 1000 may be executed as part of method 900 described above, which in turn may be executed as part of method 800 described above. Components described herein with reference to method 1000 may be the same as those described above with reference to method 800 and method 900 and may not be re-introduced.

The method at operation 1002 includes providing a 100% ventilator drive gas concentration option for delivery to an inspiratory port of an anesthesia system without mixing the ventilator drive gas with gas from a mixer. The 100 percent ventilator drive gas concentration option refers to an option that may be selected by the user (e.g., clinician) of the anesthesia machine to provide ventilator drive gas to the inspiratory port of the anesthesia machine without mixing the ventilator drive gas with gas from the gas mixer. As one example, the composition of the ventilator drive gas may be pure air, and during conditions in which the 100 percent ventilator drive gas concentration option is selected by the user, the electronic controller of the anesthesia machine may control operation of the ventilator assembly in order to provide a full the entire flow of air from the ventilator drive gas source to the inspiratory port without mixing the air with other gases from the gas mixer (e.g., the controller may adjust valves of the gas mixer in order to stop the flow of gases from the gas mixer to the inspiratory port while the ventilator drive gas is provided to the inspiratory port). Providing the full flow of the ventilator drive gas to the inspiratory port may provide for an increased flow of gas to the inspiratory port relative to conditions in which only gases from the gas mixer flow to the inspiratory port. For example, ventilator drive gases may flow to the inspiratory port with a first range of flow rates, whereas gases may flow to the inspiratory port from the gas mixer with a second range of flow rates, where the first range includes higher flow rates than the second range. As one example, the first range of flow rates may be 45 to 60 liters per minute, while the second flow range may be 0 to 15 liters per minute. Therefore, providing the ventilator drive gas to the inspiratory port may increase the rate at which gas is provided to the patient, which may increase an ease of some clinical operations such as patient intubation.

The method continues from operation 1002 to operation 1004 where the method includes determining whether the anesthesia system includes a ventilator drive gas selector. Determining whether the anesthesia machine includes a ventilator drive gas selector may include determining whether the anesthesia machine includes multi-gas unit, such as the multi-gas unit 328 described above with reference to FIG. 3. In particular, the ventilator drive gas selector may be referred to herein as a multi-gas unit. The ventilator drive gas selector may include multiple ventilator drive gas sources and may further include valves that may be controlled by the electronic controller in order to select which ventilator drive gas source provides ventilator drive gas to the bellowless plenum, inspiratory port, and other components of the anesthesia machine. For example, during a first set of conditions, the electronic controller may control valves of the ventilator drive gas selector to flow ventilator drive gas to the inspiratory port of the anesthesia machine from a first ventilator drive gas source, and during a second set of conditions, the electronic controller may control valves of the ventilator drive gas selector to flow ventilator drive gas to the inspiratory port of the anesthesia machine from a second ventilator drive gas source. The composition of the gas provided by the first ventilator drive gas source may be different from the composition of gas provided by the second ventilator drive gas source. In this way, the controller may adjust operation of the anesthesia machine based on user input to provide different ventilator drive gases to the inspiratory port, which may increase a number of gas combinations and/or ratios that may be provided to the inspiratory port.

If it is determined that the anesthesia system includes a ventilator drive gas selector at operation 1004, the method continues from operation 1004 to operation 1006 where the method includes providing a first set of selectable gas concentrations and flow rates based on the number and composition of the ventilator drive gases and the number and composition of mixer gases. The first set of selectable gas concentrations and flow rates may include a plurality of gas concentrations and flow rates that may be achieved through a combination of the ventilator drive gas with gases from the gas mixer. In some examples, the gas concentrations and flow rates that may be selected by the user may be stored within a lookup table stored in non-transitory memory of the electronic controller.

As one example, the user may select (from the first set of selectable gas concentrations) a concentration of 80% oxygen to be delivered to the inspiratory port. The user may further select (from the first set of selectable flow rates) a flow rate of 60 liters per minute of the 80% oxygen to be delivered to the inspiratory port. The selections may be made by the user via a user interface device (e.g., keyboard, touchscreen, trackball, etc.) electronically communicatively coupled with the electronic controller. As a result of the user selections, the controller may adjust operation of the anesthesia machine to combine a flow of 45 liters per minute of 100% oxygen from the ventilator drive gas source with a flow of 15 liters per minute of 100% air from the mixer gas source to form the desired mixture of 80% oxygen flowing at 60 liters per minute upstream of the inspiratory port and downstream of the bellowless plenum. The mixture may be provided to a patient through the inspiratory port via a nasal cannula, in some examples. The amount of ventilator drive gas provided to the inspiratory port relative to the amount of mixer gas provided to the inspiratory port may be referred to herein as a gas ratio of the ventilator drive gas to the mixer gas (e.g., the ventilator drive gas and the mixer gas may be provided concurrently to the inspiratory port as an inspiratory gas mixture, with the amount of ventilator drive gas to mixer gas within the inspiratory gas mixture being referred to as the gas ratio). The amount of ventilator drive gas and the amount of mixer gas may refer to moles of ventilator drive gas and moles of mixer gas, respectively, in some examples.

As another example, the user may select (from the first set of selectable gas concentrations) a concentration of 40% oxygen to be delivered to the inspiratory port. The user may further select (from the first set of selectable flow rates) a flow rate of 60 liters per minute of the 40% oxygen to be delivered to the inspiratory port. The selections may be made by the user via the user interface device. As a result of the user selections, the controller may adjust operation of the anesthesia machine to combine a flow of 45 liters per minute of 100% air from the ventilator drive gas source with a flow of 15 liters per minute of 100% oxygen from the mixer gas source to form the desired mixture of 40% oxygen flowing at 60 liters per minute upstream of the inspiratory port and downstream of the bellowless plenum. The mixture may be provided to the patient through the inspiratory port via the nasal cannula, in some examples.

In some examples, the ventilator drive gas selector may provide ventilator drive gas from multiple ventilator drive gas sources concurrently. For example, the anesthesia machine may be configured to include a first ventilator drive gas source including 100 percent oxygen and second ventilator drive gas source including 100 percent air. The electronic controller may control the valves of the ventilator drive gas selector to flow 100 percent oxygen from the first ventilator drive gas source and 100 percent air from the second ventilator drive gas source concurrently to the bellowless plenum and inspiratory port based on user input. Flowing ventilator drive gas from the first ventilator drive gas source and ventilator drive gas from the second ventilator drive gas source concurrently may include opening valves controlling the flow of gases from each ventilator drive gas source by an equal amount.

As one example, the user may select (from the first set of selectable gas concentrations) a concentration of 70% oxygen to be delivered to the inspiratory port. The user may further select (from the first set of selectable flow rates) a flow rate of 60 liters per minute of the 70% oxygen to be delivered to the inspiratory port. The selections may be made by the user via the user interface device. As a result of the user selections, the controller may adjust operation of the anesthesia machine to combine a flow of 22.5 liters per minute of 100% oxygen from a first ventilator drive gas source with a concurrent flow of 22.5 liters per minute of 100% air from a second ventilator drive gas source. The combined ventilator drive gases may be mixed with a flow of 15 liters per minute of 100% oxygen from the mixer gas source to form the desired mixture of 70% oxygen flowing at 60 liters per minute upstream of the inspiratory port and downstream of the bellowless plenum. The mixture may be provided to the patient through the inspiratory port via the nasal cannula, in some examples.

As another example, the user may select (from the first set of selectable gas concentrations) a concentration of 50% oxygen to be delivered to the inspiratory port. The user may further select (from the first set of selectable flow rates) a flow rate of 60 liters per minute of the 50% oxygen to be delivered to the inspiratory port. The selections may be made by the user via the user interface device. As a result of the user selections, the controller may adjust operation of the anesthesia machine to combine a flow of 22.5 liters per minute of 100% oxygen from a first ventilator drive gas source with a concurrent flow of 22.5 liters per minute of 100% air from a second ventilator drive gas source. The combined ventilator drive gases may be mixed with a flow of 15 liters per minute of 100% air from the mixer gas source to form the desired mixture of 50% oxygen flowing at 60 liters per minute upstream of the inspiratory port and downstream of the bellowless plenum. The mixture may be provided to the patient through the inspiratory port via the nasal cannula, in some examples. Although example selectable gas concentrations and flow rates are described above, other examples are possible.

However, if it is determined that the anesthesia system does not include a ventilator drive gas selector at operation 1004, the method continues from operation 1004 to operation 1008 where the method includes providing a second set of selectable gas concentrations and flow rates based on the composition of the ventilator drive gas and the number and concentration of mixer gases. For example, in some configurations, the anesthesia machine may not include the ventilator drive gas selector and instead may include a single ventilator drive gas source. The composition of the gas provided by the single ventilator drive gas source may be pre-determined (e.g., the ventilator drive gas source may be hard-plumbed to the anesthesia machine) and may be known by the electronic controller (e.g., the composition of the gas of the single ventilator drive gas source may be stored in non-transitory memory of the controller). In some examples, the second set of selectable gas concentrations and flow rates may be different from the first set of selectable gas concentrations and flow rates described above. For example, the selection of 80% oxygen concentration at a flow rate of 60 liters per minute and the selection of 40% oxygen concentration at a flow rate of 60 liters per minute may be included in the second set of selectable gas concentrations and flow rates as described above. However, as the anesthesia machine includes a single ventilator drive gas source, some combinations of selectable oxygen concentrations and flow rates may be dependent on the composition of the gas provided by the single ventilator drive gas source. In some examples, the user may change the composition of the ventilator drive gas source by removing and replacing cylinders of the anesthesia machine configured to provide the ventilator drive gas, which may adjust the selectable options for concentration and flow rate provided by the electronic controller.

The technical effect of flowing ventilator drive gas through the bellowless plenum to the inspiratory port of the anesthesia machine is to provide an increased range of available gas concentrations and gas flow rates of gases for inhalation by a patient.

The disclosure also provides support for a method for an anesthesia system, comprising: flowing ventilator drive gas through a bellowless plenum separating a ventilator drive gas circuit from an inspiratory gas circuit, and providing the ventilator drive gas flowing through the bellowless plenum to an inspiratory port of the inspiratory gas circuit. In a first example of the method, the method further comprises: flowing the ventilator drive gas through the bellowless plenum to a nasal cannula. In a second example of the method, optionally including the first example, the method further comprises: flowing the ventilator drive gas provided to the inspiratory port through a multi-outlet gas diverter arranged upstream of the nasal cannula. In a third example of the method, optionally including one or both of the first and second examples, providing the ventilator drive gas flowing through the bellowless plenum to the inspiratory port of the inspiratory gas circuit includes: mixing the ventilator drive gas with mixer gas from a mixer gas source to form an inspiratory gas mixture, and delivering the inspiratory gas mixture to the inspiratory port. In a fourth example of the method, optionally including one or more or each of the first through third examples, combining the ventilator drive gas with the mixer gas occurs downstream of the bellowless plenum along a flow path from the bellowless plenum to the inspiratory port. In a fifth example of the method, optionally including one or more or each of the first through fourth examples, the method further comprises: adjusting a gas ratio of the inspiratory gas mixture, where the gas ratio is a ratio of the ventilator drive gas to the mixer gas. In a sixth example of the method, optionally including one or more or each of the first through fifth examples, the method further comprises: adjusting an amount of the mixer gas within the inspiratory gas mixture based on a composition of the ventilator drive gas.

The disclosure also provides support for a method for an anesthesia system, comprising: flowing ventilator drive gas from a ventilator drive gas source to a patient inspiratory port via a bellowless plenum separating the ventilator drive gas source from a mixer gas source, and adjusting a flow rate of the ventilator drive gas based on the mixer gas source. In a first example of the method, the method further comprises: forming a mixture of the ventilator drive gas and a mixer gas from the mixer gas source downstream of the bellowless plenum and upstream of the patient inspiratory port. In a second example of the method, optionally including the first example, the method further comprises: flowing the mixture of the ventilator drive gas and the mixer gas to a nasal cannula coupled to the patient inspiratory port. In a third example of the method, optionally including one or both of the first and second examples, the method further comprises: adjusting the mixture of the ventilator drive gas and the mixer gas based on a selection input via a user interface device. In a fourth example of the method, optionally including one or more or each of the first through third examples, adjusting the mixture of the ventilator drive gas and the mixer gas based on the selection input via the user interface device includes adjusting a ratio of the ventilator drive gas to the mixer gas. In a fifth example of the method, optionally including one or more or each of the first through fourth examples, adjusting the ratio of the ventilator drive gas to the mixer gas includes adjusting a flow rate of the ventilator drive gas or a flow rate of the mixer gas. In a sixth example of the method, optionally including one or more or each of the first through fifth examples, adjusting the flow rate of the ventilator drive gas based on the mixer gas source includes setting the flow rate of the ventilator drive gas according to a composition of a mixer gas of the mixer gas source. In a seventh example of the method, optionally including one or more or each of the first through sixth examples, the method further comprises: after flowing the ventilator drive gas from the ventilator drive gas source to the patient inspiratory port via the bellowless plenum, controlling a ventilator drive gas selector to flow a second ventilator drive gas from a second ventilator drive gas source to the patient inspiratory port via the bellowless plenum. In a eighth example of the method, optionally including one or more or each of the first through seventh examples, the method further comprises:, while flowing the ventilator drive gas to the patient inspiratory port via the bellowless plenum, maintaining an exhalation valve arranged between the ventilator drive gas source and the bellowless plenum in a closed position.

The disclosure also provides support for an anesthesia system, comprising: a ventilator drive gas source, a patient inspiratory port, a bellowless plenum separating the ventilator drive gas source from the patient inspiratory port and including a first port fluidly coupled to the ventilator drive gas source and a second port fluidly coupled to the patient inspiratory port, and an electronic controller including instructions stored in non-transitory memory that when executed, cause the electronic controller to: flow a ventilator drive gas from the ventilator drive gas source through the bellowless plenum to the patient inspiratory port. In a first example of the system, while flowing the ventilator drive gas from the ventilator drive gas source through the bellowless plenum to the patient inspiratory port, an entire amount of ventilator drive gas output by the ventilator drive gas source flows through the bellowless plenum to the patient inspiratory port. In a second example of the system, optionally including the first example, the system further comprises: instructions stored in the non-transitory memory that when executed, cause the electronic controller to: adjust a flow rate of the ventilator drive gas based on a mixer gas source fluidly coupled to the patient inspiratory port via an inspiratory gas circuit. In a third example of the system, optionally including one or both of the first and second examples, the system further comprises: a ventilator drive gas selector, and instructions stored in the non-transitory memory of the electronic controller than when executed, cause the electronic controller to: control the ventilator drive gas selector to selectably fluidly couple a second ventilator drive gas source to the patient inspiratory port via the bellowless plenum.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative only and should not be construed to be limiting in any manner.

## Claims

1. A method (800) for an anesthesia system (600), comprising:
flowing ventilator drive gas through a bellowless plenum (502) separating a ventilator drive gas circuit (352) from an inspiratory gas circuit (500); and
providing the ventilator drive gas flowing through the bellowless plenum (502) to an inspiratory port (518) of the inspiratory gas circuit (500).

2. The method (800) of claim 1, further comprising: flowing the ventilator drive gas through the bellowless plenum (502) to a nasal cannula (608).

3. The method (800) of claim 2, further comprising: flowing the ventilator drive gas provided to the inspiratory port (518) through a multi-outlet gas diverter (712) arranged upstream of the nasal cannula (608).

4. The method (800) of claim 1, wherein providing the ventilator drive gas flowing through the bellowless plenum (502) to the inspiratory port (518) of the inspiratory gas circuit (500) includes:
mixing the ventilator drive gas with mixer gas from a mixer gas source (400) to form an inspiratory gas mixture; and
delivering the inspiratory gas mixture to the inspiratory port (518).

5. The method (800) of claim 4, wherein combining the ventilator drive gas with the mixer gas occurs downstream of the bellowless plenum (502) along a flow path (614) from the bellowless plenum (502) to the inspiratory port (518).

6. The method (800) of claim 4, further comprising adjusting a gas ratio of the inspiratory gas mixture, where the gas ratio is a ratio of the ventilator drive gas to the mixer gas.

7. The method (800) of claim 4, further comprising adjusting an amount of the mixer gas within the inspiratory gas mixture based on a composition of the ventilator drive gas.

8. An anesthesia system (600), comprising:
an electronic controller (240) including instructions stored in non-transitory memory that when executed, cause the electronic controller (240) to:
flow ventilator drive gas from a ventilator drive gas source (341) to a patient inspiratory port (518) via a bellowless plenum (502) separating the ventilator drive gas source (341) from a mixer gas source (400); and
adjust a flow rate of the ventilator drive gas based on the mixer gas source (400).

9. The anesthesia system (600) of claim 8, further comprising instructions stored in non-transitory memory of the electronic controller (240) that when executed, cause the electronic controller (240) to:
form a mixture of the ventilator drive gas and a mixer gas from the mixer gas source (400) downstream of the bellowless plenum (502) and upstream of the patient inspiratory port (518).

10. The anesthesia system (600) of claim 9, further comprising instructions stored in non-transitory memory of the electronic controller (240) that when executed, cause the electronic controller (240) to:
flow the mixture of the ventilator drive gas and the mixer gas to a nasal cannula (608) coupled to the patient inspiratory port (518).

11. The anesthesia system (600) of claim 9, further comprising instructions stored in non-transitory memory of the electronic controller (240) that when executed, cause the electronic controller (240) to:
adjust the mixture of the ventilator drive gas and the mixer gas based on a selection input via a user interface device.

12. The anesthesia system (600) of claim 11, wherein adjusting the mixture of the ventilator drive gas and the mixer gas based on the selection input via the user interface device includes adjusting a ratio of the ventilator drive gas to the mixer gas.

13. The anesthesia system (600) of claim 8, wherein adjusting the flow rate of the ventilator drive gas based on the mixer gas source (400) includes setting the flow rate of the ventilator drive gas according to a composition of a mixer gas of the mixer gas source (400).

14. The anesthesia system (600) of claim 8, further comprising instructions stored in non-transitory memory of the electronic controller (240) that when executed, cause the electronic controller (240) to:
control a ventilator drive gas selector (328) to flow a second ventilator drive gas from a second ventilator drive gas source (339) to the patient inspiratory port (518) via the bellowless plenum (502) after flowing the ventilator drive gas from the ventilator drive gas source (341) to the patient inspiratory port (518) via the bellowless plenum (502).

15. The anesthesia system (600) of claim 8, further comprising instructions stored in non-transitory memory of the electronic controller (240) that when executed, cause the electronic controller (240) to:
maintain an exhalation valve (346) arranged between the ventilator drive gas source (341) and the bellowless plenum (502) in a closed position while flowing the ventilator drive gas to the patient inspiratory port (518) via the bellowless plenum (502).
